Europäisches Patentamt
European Patent Office
Office européen des brevets

(11) Publication number: **0 042 669**
**A2**

(12) # EUROPEAN PATENT APPLICATION

(21) Application number: 81302322.3

(22) Date of filing: 26.05.81

(51) Int. Cl.³: C 07 D 498/00
C 07 D 239/50, C 07 D 239/48
C 07 D 251/54, C 07 D 251/48
A 23 K 1/16
//(C07D498/00, 271/00, 251/00),
(C07D498/00, 271/00, 239/00)

(30) Priority: 23.06.80 US 161943
23.06.80 US 161944
23.06.80 US 161945
23.06.80 US 161946
23.06.80 US 161976
14.04.81 US 249762

(43) Date of publication of application:
30.12.81 Bulletin 81/52

(84) Designated Contracting States:
BE CH DE FR GB IT LI NL SE

(71) Applicant: THE UPJOHN COMPANY
301 Henrietta Street
Kalamazoo, Michigan 49001(US)

(72) Inventor: Degeeter, Melvin Joseph
6109 Ormada Street
Kalamazoo Michigan(US)

(72) Inventor: Mccall, John Michael
3822 Edinburgh
Kalamazoo Michigan(US)

(72) Inventor: Teagarden, Dirk Lowell
2911 South Rose Street
Kalamazoo, Michigan(US)

(74) Representative: Perry, Robert Edward et al,
GILL JENNINGS & EVERY 53-64 Chancery Lane
London WC2A 1HN(GB)

(54) **Compounds and compositions for use in animal feeds.**

(57) Compounds of formula Ia or Ib

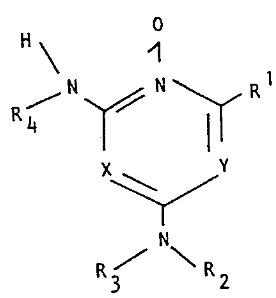

1a

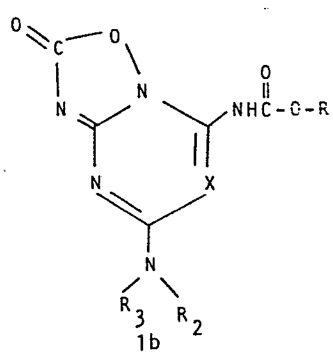

1b

wherein R is alkyl; $R_1$ is alkyl, alkoxycarbonylamino or acylamino; $NR_2R_3$ is mono- or di-substituted amino or a heterocyclic ring; $R_4$ is H, alkoxycarbonyl, alkenyl, cycloalkyl or acyl; X is N or CH; and Y is N, CH, CBr, CCl or Calkyl, can be administered to healthy animals to increase weight gain or meat, egg or wool production. For example, they may be administered in the form of a long-acting composition.

EP 0 042 669 A2

COMPOUNDS AND COMPOSITIONS FOR USE IN ANIMAL FEEDS

This invention relates to the use of diazine, triazine and pyridine compounds in increasing production in healthy animals, and to compositions for use as animal feeds. Suitable animals are meat-producing, milk-producing, wool-producing or egg-laying.

It is known that meat-producing animals can gain weight, and show increased weight gain, when various compounds such as vitamins, minerals, estrogens, antibiotics and/or tranquillizers are added to the animal's diet.

It has now been discovered that compounds of formulae Ia and Ib can be administered to appropriate animals to increase the rate or amount of weight gain, increase milk production or wool production or the rate of egg-laying, or increase feed efficiency. The active compounds have the formulae

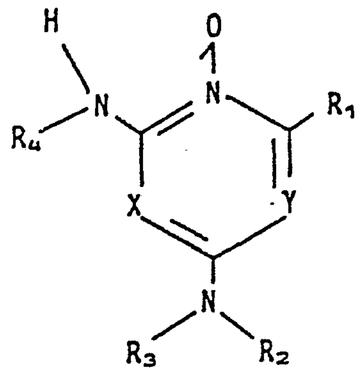

Ia

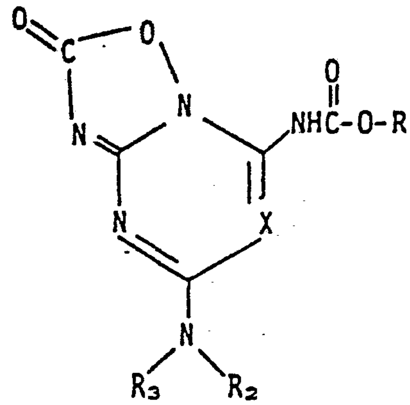

Ib

wherein

X is N or CH;

Y is N or $CR_5$;

R is alkyl of from 1 to 8 carbon atoms, inclusive, including isomeric forms thereof;

$R_1$ is R or

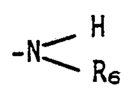

$R_2$ and $R_3$ are the same or different and are hydrogen, provided that $R_2$ and $R_3$ are not both hydrogen, R, cycloalkyl of from 3 to 8 carbon atoms, inclusive, alkyl substituted cycloalkyl of the formula

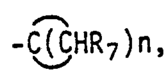

alkenyl of from 2 to 8 carbon atoms, inclusive, including isomeric forms thereof, aralkyl wherein

Ar is phenyl or substituted phenyl wherein 1 or 2 hydrogens are replaced with chlorine, fluorine, bromine, iodine, R, -OR, or $CF_3$, and the substituents can be the same or different, and alkyl is from 1 to 4 carbon atoms, inclusive, including isomeric forms thereof, and $R_2$ and $R_3$ taken together with -N< is a heterocyclic moiety of from 3 to 8, inclusive, ring atoms and 1 or 2 hetero atoms selected from the group consisting of nitrogen, oxygen or sulfur or a substituted heterocyclic moiety wherein 0, 1, 2, or 3 of the carbon atoms of the

heterocycle are substituted with R;

$R_4$ is hydrogen, $-\overset{O}{\overset{\|}{C}}-O-R$, alkenyl of from 2 to 8 carbon atoms, inclusive, including isomeric forms thereof, cycloalkyl of from 3 to 7 carbon atoms, inclusive, or lower acyl wherein acyl is up to and including 5 carbon atoms;

$R_5$ is hydrogen, R, bromo or chloro;

$R_6$ is hydrogen, $-\overset{O}{\overset{\|}{C}}-O-R$ or lower acyl;

$R_7$ is hydrogen or alkyl of from 1 to 5 carbon atoms, inclusive, including isomeric forms thereof;

N is an integer of from 2 to 7, inclusive.

The compounds of the Formula Ia exist in tautomeric forms. It is to be understood that the compounds of this invention are likely to be mixtures of tautomeric forms, the compositions of which are dependent on such factors as the nature of the substituent groups and the environment. In some instances, one form or another may predominate.

Diazine compounds of the Formulas Ia can be prepared by methods disclosed in United States Patents 3,910,928 (October 7, 1975), 4,032,559 (June 28, 1977).

Pyridine compounds of the Formula Ia can be prepared by methods disclosed in United States Patent 4,021,562.

Triazine compounds of the Formula Ia can be prepared by methods disclosed in United States Patents 3,475,430, 3,270,014, 3,270,018, and 3,270,015.

Triazine compounds of the Formula Ib can be prepared by methods disclosed in Belgium Patent 863,608, issued March 18, 1978, and United States Patent 4,150,131 substituting a triazine compound for the diazene of the patent.

Diazine compounds of the Formula Ib can be prepared by methods disclosed in Belgium Patent 863,608, issued March 18, 1978, and United States Patents 4,175,190 and 4,150,131.

The compounds of Formulas Ia and Ib are amines, and exist in the non-protonated or free base form, or in the protonated or acid addition salt form, depending on the pH of the environment. They form stable protonates, i.e., mono- or diacid addition salts, on treatment with suitable acids, for example, hydrochloric, hydrobromic, sulfuric, phosphoric, nitric, acetic, benzoic, salicylic, glycolic, succinic,

nicotinic, tartaric, maleic, malic, pamoic, methanesulfonic, cyclohexanesulfamic, picric, and lactic acids, and the like.

Examples of alkyl are methyl, ethyl, propyl, butyl, pentyl, hexyl, heptyl, octyl, and isomeric forms thereof. Examples of alkenyl are allyl, 1-methylallyl, 2-methylallyl (methallyl), 2-butenyl (crotyl), 3-butenyl, 1,2-dimethylallyl, 1,1-dimethylallyl, 2-ethylallyl, 1-methyl-2-butenyl, 2-methyl-2-butenyl, 3-methyl-2-butenyl, 3-pentenyl, 2,3-dimethyl-2-butenyl, 1,1,2-trimethylallyl, 1,3-dimethyl-2-butenyl, 1-ethyl-2-butenyl, 4-methyl-2-pentenyl, 2-ethyl-2-pentenyl, 4,4-dimethyl-2-pentenyl, 2-heptenyl, 2-octenyl, 5-octenyl, 1,4-dimethyl-4-hexenyl, and the like. Examples of cycloalkyl are cyclopropyl, 2-methylcyclopropyl, 2,2-dimethylcyclopropyl, 2,3-diethylcyclopropyl, 2-butylcyclopropyl, cyclobutyl, 2-methylcyclobutyl, 3-propylcyclobutyl, 2,3,4-triethylcyclobutyl, cyclopentyl, 2,2-dimethylcyclopentyl, 3-pentylcyclopentyl, 3-tert-butylcyclopentyl, cyclohexyl, 4-tert-butylcyclohexyl, 3-isopropylcyclohexyl, 2,2-dimethylcyclohexyl, cycloheptyl, cyclooctyl and the like. Examples of aralkyl are benzyl, phenethyl, 1-phenylethyl, 2-phenylpropyl, 4-phenylbutyl, 5-phenyl-2-methylpentyl, 1-naphthylmethyl, 2-(1-naphthyl)ethyl, 2-(2-naphthyl)ethyl and the like. Examples of acyl are acetyl, propionyl, butanoyl, pentanoyl and the like. Examples of alkoxycarbonyl are carbomethoxy, carboethoxy and the like. Examples of heterocyclic moieties of the present invention are piperidino, pyrrolidinyl, morpholino, 2,4,4-trimethylazetidinyl, 2,3,4-trimethylazetidinyl, 2-methylpyrrolidinyl, 3-butylpyrrolidinyl, 2-isohexylpyrrolidinyl, 2,3-dimethylpyrrolidinyl, 2,2-dimethylpyrrolidinyl, 2,5-diethylpyrrolidinyl, 3-tert-butylpyrrolidinyl, 2,3,5-trimethylpyrrolidinyl, 3,4-dioctylpyrrolidinyl, 2-methylpiperidino, 3-methylpiperidino, 4-methylpiperidino, 3-isopropylpiperidino, 4-tert-butylpiperidino, 2-methyl-5-ethylpiperidino, 3,5-dipentylpiperidino, 2,4,6-trimethylpiperidino, 2,6-dimethylpiperidino, 2,6-dimethyl-4-octylpiperidino, 2,3,5-triethylpiperidino, 2-ethylhexahydroazepinyl, 4-tert-butylhexahydroazepinyl, 3-heptylhexahydroazepinyl, 2,4-dimethylhexahydroazepinyl, 3,3-dimethylhexahydroazepinyl, 2,4,6-tripropylhexahydroazepinyl, 2-methylheptamethylenimino, 5-butylheptamethylenimino, 2,4-diisopropylheptamethylenimino, 3,3-diethylheptamethylenimino, 2,5,8-trimethylheptamethylenimino, 3-methyloctamethylenimino, 2,9-diethyloctamethylenimino, 4-isooctyloctamethylenimino, 2-ethylmorpholino, 2-methyl-5-ethylmorpholino, 3,3-di-

methylmorpholino, 2,6-di-tert-butylmorpholino, 4-methylpiperazinyl, 4-isopropylpiperazinyl, 2-methylaziridinyl, 2-ethylaziridinyl, 2-butylaziridinyl, 2,3-dimethylaziridinyl, 2,2-dimethylaziridinyl, 2-methylazetidinyl, 3-methylazetidinyl, 2-octylazetidinyl, 2,2-dimethylazetidinyl, 3,3-diethylazetidinyl and the like.

Unless otherwise specified, all percentages are given on a weight-to-weight basis.

Administration of the compositions of the present invention can commence for birds shortly after hatching and in the case of mammals, during the creep-feeding period of suckling animals when they are starting on solid food and, of course, after weaning. Feeding of the compositions is continued throughout the growing period, lactation period, or egg-laying period. In addition to feeding the active ingredient in combination with the feed, the active compounds can alternatively be administered in combination with the animals drinking water or in combination with a pharmaceutical carrier by injection or implantation.

The total concentration of the compound of the Formula Ia or Ib in the feed composition is determined with regard to the species of animal, sex, age, weight, and average amount of feed consumed daily. Preferably the compound of the Formula I is employed in the finished feed that will supply the animal with a daily intake of from about 0.02 mg to about 200 mg per head, per day.

The following table illustrates the range of compound of Formula Ia or Ib in milligrams daily dose, per head, per day for representative animals.

| Animal | Range Daily Dose/Head, mg | Preferred Daily Dose, mg |
|---|---|---|
| Swine (birth to 8 weeks) | 0.5 - 20.0 | 10.0 |
| Swine (18 to 90 kg.) | 1.0 - 140.0 | 10.0 |
| Chickens (growing 0-8 weeks) | 0.02 - 2.0 | 0.05 |

| | | |
|---|---|---|
| Hens (laying) | 0.1 - 2.0 | 1.0 |
| Turkeys (growing 0-24 weeks) | 0.1 - 5.0 | 2.0 |
| Beef Cattle (fattening) | 0.5 - 50.0 | 10 |
| Calves (0-12 weeks) | 1.0 - 40.0 | 10 |
| Dairy Cattle (lactation) | 5.0 - 200.0 | 10 |
| Lambs (fattening) | 1.0 - 20.0 | 10 |

The foregoing dosages can generally be accomplished by providing from about 50 mg to about 20,000 mg of a compound of the Formula Ia or Ib per ton of finished feed.

Advantageously a compound of the Formula Ia or Ib is supplied in the form of a liquid or solid premix wherein the concentration is 100-2,000 times greater than the desired final concentration in the feed. For example, the compound of Formula Ia or Ib can be dissolved or suspended in a fluid vehicle such as corn oil, cottonseed oil, molasses, distillers solubles and the like to prepare a fluid premix. Alternatively, a solid premix can be prepared by mixing a compound of the Formula Ia or Ib with an edible solid diluent such as sucrose, lactose, starch, corn meal, flour, calcium carbonate, soybean meal and the like.

<u>Example 1</u>

A diet for fattening lambs is prepared from the following types and amounts of ingredients:

| | |
|---|---|
| Ground ear corn | 82.35% |
| Alfalfa meal | 10.0 % |

| | |
|---|---|
| Soybean oil meal 44% | 7.0 % |
| Ground limestone | 0.3 % |
| Salt | 0.3 % |
| Trace mineral mixture[1] | 0.05% |

[1]Contains the following percent of minerals: Mn, 12; Co, 0.08; Fe, 5.0; Cu, 0.4; I, 0.24; Zn, 0.7.

The above feed to be mixed, pelleted and offered to fattening lambs free-choice in conjunction with hay.

To 999 parts of the preceding feed is added 1 part of a premix composition prepared by mixing 7 gm of 2,4-diamino-6-piperidino-pyrimidine-3-oxide with sufficient corn meal to make one pound.

The feeding composition so prepared supplies 7.0 mg of 2,4-diamino-6-piperidinopyrimidine-3-oxide per pound or 15.4 parts per million.

The foregoing composition is usefully fed to lambs for increased rate of weight gain and improved utilization of feed.

Example 2

A chicken feed for broilers is prepared from the following types and amounts of ingredients:

| | |
|---|---|
| Yellow corn meal | 67.35% |
| Soybean oil meal | 24.00% |
| Menhaden fish meal | 6.00% |
| Steamed bone meal | 1.00% |
| Ground limestone | 1.00% |
| Iodized salt | .34% |
| 25% Choline chloride | .13% |

| | |
|---|---|
| Vitamin $B_{12}$ supplement (6 mg/lb) | .10% |
| Manganese sulfate | .02% |
| Supplemental vitamin mix[1] | .06% |

[1]Consisting of 16.0 gm Vitamin A supplement (10 units/mg); 3.6 gm Vitamin $D_3$ supplement (15,000 units/gm); 7.1 gm riboflavin supplement (1 gm riboflavin per ounce); 500 mg niacin.

To 999 parts of the preceding feed is added 1 part of a premix composition prepared by mixing 0.38 gm of 2,4-diamino-6-piperidino-pyrimidine-3-oxide with sufficient soybean mill feed to make 1 pound.

The feeding composition so prepared supplies .38 mg of 2,4-diamino-6-piperidinopyrimidine-3-oxide per pound, or about .83 parts per million.

The foregoing composition is usefully fed to chickens for increased rate of weight gain and improved utilization of feed. Similarly the composition can be fed to turkeys, ducks and geese.

Example 3

A fattening feed for 800 pound yearling cattle is prepared from the following types and amounts of ingredients:

| | |
|---|---|
| Ground ear corn | 89.75% |
| Soybean oil meal, 44% | 9.0 % |
| Ground limestone | 0.7 % |
| Salt | 0.5 % |
| Trace mineral mixture[1] | 0.05% |

[1]Contains the following percent of minerals: Mn, 12, Co, 0.08; Fe, 5.0; Cu, 0.4; I, 0.24; Zn, 0.7.

To 999 parts of the preceding feed is added 1 part of a premix composition prepared by mixing 0.5 gm of 2,4-diamino-6-piperidino-

pyrimidine-3-oxide with sufficient wheat flour to make 1 pound.

The feeding composition so prepared supplies 0.5 mg of 2,4-diamino-6-piperidinopyrimidine-3-oxide per pound, or about 1.10 parts per million.

Cattle are to receive the foregoing feed ad libitum together with 5 lbs. of hay, per head, per day and when so fed have an increased rate of weight gain and improved utilization of feed.

Example 4

A swine diet for growing hogs of 40 to 100 pounds body weight is prepared from the following types and amounts of ingredients:

| | |
|---|---|
| Corn, ground | 78.15% |
| Soybean oil meal, 44% | 17.0 % |
| Meat and bone scraps, 50% | 3.0 % |
| Oyster shell flour | 0.4 % |
| Bone meal | 0.5 % |
| Salt | 0.5 % |
| Trace mineral mixture | 0.05% |
| Zinc oxide | 0.01% |
| Vitamin A and D Supplement[2] | 0.25% |
| Vitamin Supplement [3] | 0.05% |
| Vitamin $B_{12}$ Supplement [4] | 0.09% |

[1]Contains the following % of minerals: Mn, 12; Co, 0.08; Fe, 5.0; Cu, 0.4; I, 0.24; Zn, 0.7.

[2]Contains 300 USP units $D_2$/Gm and 1500 IU of A/Gm

[3]Contains per lb: Riboflavin, 2000 mg; calcium pantothenate, 4000 mg; niacin, 9000 mg; and choline chloride 10,000 mg.

[4]Contains 6 mg Vitamin $B_{12}$ per lb.

To 999 parts of the preceding feed is added 1 part of a premix composition prepared by mixing 2 g of 2,4-diamino-6-piperidinopyrimidine-3-oxide with sufficient ground limestone to make one pound.

The feeding composition so prepared supplies 2 mg of 2,4-diamino-6-piperidinopyrimidine-3-oxide per pound or about 4.4 parts per million.

The foregoing composition is usefully fed to hogs for increased

rate of weight gain and improved utilization of feed.

Example 5

A regimen of 2,4-diamino-6-piperidinopyrimidine-3-oxide in water is prepared simply by adding the compound to the drinking water as specified below. The animals are allowed to ingest the water on an *ad lib* basis.

| | | mg/l |
|---|---|---|
| Swine | Birth to 8 weeks | 2.5 |
| Swine | 18-90 kg. | 7.0 |
| Chickens | 0-8 weeks | 0.3 |
| Hens | | 5.0 |
| Turkeys | 0-24 weeks | 3.2 |
| Beef cattle | | 0.2 |
| Calves | 0-12 weeks | 1.0 |
| Dairy cattle | | 0.9 |
| Lambs | | 5.0 |

Example 6

Following the procedure of the preceding Examples 1 to 5, inclusive, animal feeds are similarly prepared substituting equimolar amounts of:

2,4-diamino-6-pyrrolidinopyrimidine-3-oxide,
2,4-diamino-6-diethylaminopyrimidine-3-oxide,
2,4-diamino-6-(2-methylpiperidino)pyrimidine-3-oxide,
2-amino-4-methyl-6-pyrrolidinopyrimidine-3-oxide,
2-amino-4-methyl-6-piperidinopyrimidine-3-oxide,
2,4-diamino-6-dialkylaminopyrimidine-3-oxide,
2,4-diamino-6-piperidino-S-triazine-3-oxide,
2,4-diamino-6-pyrrolidino-S-triazine-3-oxide,
2-amino-4-methyl-6-pyrrolidino-S-triazine-3-oxide,
2,4-diamino-6-diethylamino-S-triazine-3-oxide,
2,4-diamino-6-(N,N-dialkylamino)-S-triazine-3-oxide,
2,6-diamino-4-pyrrolidinopyridine-1-oxide,
2,6-diamino-4-diethylaminopyridine-1-oxide,
2,6-diamino-4-(2-methylpiperidino)pyridine-1-oxide,

2-amino-6-methyl-4-pyrrolidinopyridine-1-oxide,

2-amino-6-methyl-4-piperidinopyrimidine-1-oxide,

2,6-diamino-4-dialkylaminopyrimidine-1-oxide,

2,6-diamino-3-piperidinopyrimidine-1-oxide,

ethyl[2-oxo-5-(1-piperidinyl)-2H-[1,2,4] oxadiazolo-[2,3-a]-pyrimidin-7-yl]carbamate,

ethyl[5-[3,6-dihydro-1(2H)-pyridyl]-2-oxo-2H-[1,2,4]-oxadiazolo-[2,3-a]pyrimidin-7-yl]carbamate,

butyl[5-[3,6-dihydro-1(2H)-pyridyl]-2-oxo-2H-[1,2,4]oxadiazolo-[2,3-a]-pyrimidin-7-yl]carbamate,

ethyl[5-dialkylamino-2-oxo-2H-[1,2,4]-oxadiazolo[2,3-a]-pyrimidin-7-yl]carbamate,

ethyl[5-diethylamino-2-oxo-2H-[1,2,4]oxadiazolo[2,3-a][1,3,5]-triazin-7-yl]carbamate;

ethyl[5-diallylamino-2-oxo-2H-[1,2,4]oxadiazolo[2,3-a][1,3,5]-triazin-7-yl]carbamate,

ethyl[5-[3,6-dihydro-1(2H)-pyridyl]2-ox-2H-[1,2,4]oxadiazolo-[2,3-a][1,3,5]triazin-7-yl]carbamate, and

ethyl[2-oxo-5-(1-piperidinyl)-2H-[1,2,4]oxadiazolo[2,3-a]-[1,3,5-]triazin-7-yl]carbamate.

for the 2,4-diamino-6-piperidinopyrimidine-3-oxide of the example.

An added advantage was observed in the feeding of sheep. The sheep produced an increased amount of wool. This increase in wool production was attributable to the increase in body size (and weight).

In addition to the daily administration of a compound of the formulas Ia and Ib, in association with the animal feed or drinking water, daily administration can be accomplished by incorporation of the active compound in a salt block in an amount calculated to provide the active compound based upon the daily salt intake of the animal.

In animals that are on open range or for whom daily administration is inconvenient a long active carrier can be prepared for the active compound. Long acting forms can be implants which are placed under the animals skin, injectable fluids or in the case of ruminants, an oral bolus.

Implants are prepared for 30 or 90 day administration incorporating 30 to 90 times the daily dose in an inert carrier material. The carrier material can be non-degradable by the animals body, e.g. silicone rubber, polyethylene or hydrophilic polymers such as hydron, or

degradable polymers such as polyactic acid, polyacetic acid/polyglycolic acid copolymer, poly(ortho-ester), poly (E-caprolactone) or polyglutamic acid. Implants are conveniently implanted in the neck of the chicken, the dewlap of the calf, ear of the pig, or flank of goats and lambs.

Long acting (90 day) injectable can be prepared incorporated 90 times the daily dose of a compound of the Formula Ia or 1p preferably a water insoluble derivatives such as the acyl diacyl or carbamate form in combination with a sterile injectable fluid carrier such as water, polyethylene glycol 400, propylene glycol or water.

Long acting orally administered forms, e.g., a bolus can be prepared for ruminant animals. The bolus can be prepared by the same materials as the implant but is made in a much larger size and advantageously has added excipients to increase the specific gravity of the devise. The bolus when administered remains in the reticulum or rumen of the animal and releases the drug over the desired period of time. Excipients to increase specific gravity can be for example, iron pellets, calcium sulfate dihydrate portland cement or plaster of paris.

<u>Example 7</u> Salt Block

Salts blocks are prepared for cattle containing for 0.001 to 0.04 percent w/w with 0.004 percent w/w being preferred. The cattle are allowed free access to the salt block for self administration <u>ad libitum</u>.

Salt blocks for sheep are prepared in a concentration of from 0.0024 to 0.048 percent w/w with 0.024 being preferred.

<u>Example 8</u> Long Acting Injectable Fluid

A long acting injectable fluid is prepared by the following types and amount of ingredients:

| | |
|---|---|
| 2-Amino-4-valeramido-6-piperidinopyrimidine-3-oxide | 1.8 gram |
| Benzyl alcohol | 0.1 gram |
| Carboxymethylcellulose | 0.1 gram |
| NaCl | 0.09 gram |
| Tween 80 | 0.1 gram |
| Propylene glycol q.s. | 10. ml |

The ingredients are mixed under aseptic conditions.

The composition when injected, 5 ml, into calves, beef or dairy

cattle or lambs provides 10 mg of drug 1 day for 90 days.

Example 9 Inplant

A silicone rubber implant is prepared for the following types and amounts of ingredients.

| | |
|---|---|
| 2,4-Diamino-6-piperidinopyrimidine-3-oxide | 0.72 gram |
| Stannous octoate | 0.26 gram |
| Dimethylpolysiloxane (Dow Corning Silastic 382) | 99. gram |

The ingredients are mixed well and placed in a cyclindrical mold and cured.

The silastic device is implanted on the dewlap of a calf or flank of a sheep or goat for 90 days of release.

For pigs the active compound is increased to 3.6 gram and the device placed in the tissue under the ear.

Example 10 Bolus for Ruminants

(A) A thirty day bolus is prepared for the following types and amount of ingredients.

| | |
|---|---|
| 2,4-Diamino-6-piperidinopyrimidine-3-oxide | .3 gram |
| Mg stearate | .5 gram |
| $CaSO_4$ dihydrate q.s. | 100.0 gram |

The bolus is prepared by mixing the ingredients together and compression. The bolus provides 30 days treatment for goats, sheep or cattle.

(B) 90 Day bolus

| | |
|---|---|
| 2,4-Diamino-6-piperidinopyrimidine-3-oxide | .9 gram |
| Mg stearate | .5 gram |
| $CaSO_4$ dihydrate q.s. | 100.0 gram |

The bolus is prepared by mixing the ingredients and compression. The bolus provides 90 days treatment for goats, sheep or cattle.

CLAIMS

1. A compound, for increased production in healthy animals, of formula Ia or Ib

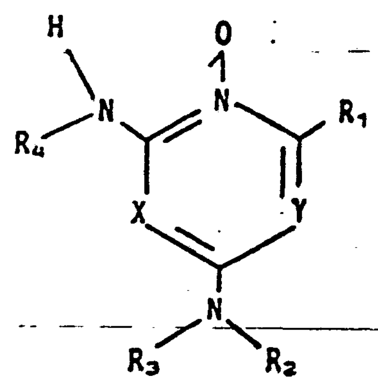

Ia

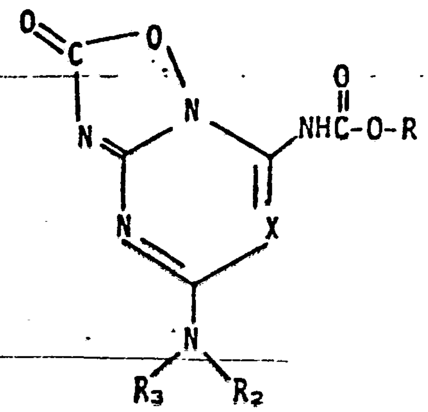

Ib

wherein R is $C_{1-8}$ alkyl;

$R_1$ is $C_{1-8}$ alkyl, $-NH_2$, ($C_{1-8}$ alkoxy)carbonyl-amino or $C_{1-8}$ acylamino;

either $R_2$ and $R_3$ are independently selected from hydrogen, $C_{1-8}$ alkyl, $C_{3-8}$ cycloalkyl, $C_{2-8}$ alkenyl, alkyl-substituted cycloalkyl of the formula $-C(CHR_7)_n$ in which $R_7$ is hydrogen or $C_{1-5}$ alkyl and n is an integer of from 2 to 7, or optionally substituted phenyl($C_{1-4}$ alkyl) in which there may be one or two substituents independently selected from halogen, $C_{1-8}$ alkyl, $C_{1-8}$ alkoxy and $-CF_3$, provided that $R_2$ and $R_3$ are not both hydrogen;

or $NR_2R_3$ is an optionally substituted heterocyclic ring having 3 to 8 ring atoms including zero, 1 or 2 further hetero atoms selected from nitrogen, oxygen and sulfur, in which there may be up to 3 $C_{1-8}$ alkyl substituents;

$R_4$ is hydrogen, ($C_{1-8}$ alkoxy)carbonyl, $C_{2-8}$ alkenyl, $C_{3-7}$ cycloalkyl or $C_{1-5}$ acyl;

X is N or CH; and

Y is N, CH, CBr, CCl or C($C_{1-8}$ alkyl).

2. A veterinary composition comprising a compound of formula Ia or Ib as defined in claim 1, in association with a veterinary carrier.

3. A composition according to claim 2, in the form of a solid animal feed.

4. A composition according to claim 2, in which the carrier comprises drinking water for an animal or animals to which the composition is to be administered.

5. A composition according to claim 2, in which the carrier comprises a salt ration for an animal or animals to which the composition is to be administered.

6. A composition according to claim 2, for long-acting use, in which the carrier comprises a bolus, implant or sterile injectable vehicle.

7. A composition according to any of claims 2 to 6, which comprises from 5g to 40kg of the compound per 1000kg of the carrier.

8. A process for obtaining increased production in healthy animals, which comprises administering to the animal a compound or composition according to any preceding claim.